(19) Europäisches Patentamt
European Patent Office
Office européen des brevets

(11) Publication number: **0 209 275**
**B1**

(12) # EUROPEAN PATENT SPECIFICATION

(45) Date of publication of patent specification: **19.10.88**

(51) Int. Cl.⁴: **C 07 C 91/28**, A 61 K 31/135

(21) Application number: **86304916.9**

(22) Date of filing: **25.06.86**

(54) **Benz-trisubstituted 2-aminotetralins.**

(30) Priority: **26.06.85 US 749030**

(43) Date of publication of application:
**21.01.87 Bulletin 87/04**

(45) Publication of the grant of the patent:
**19.10.88 Bulletin 88/42**

(84) Designated contracting states:
**AT BE CH DE FR GB IT LI LU NL SE**

(56) References cited:
**US-A- 3 969 316**
**JOURNAL OF MEDICINAL CHEMISTRY, vol. 22, no. 11, November 1979, pages 1323-1329, American Chemical Society, US; J.Z. GINOS et al.: "Structure-activity relationships of N-substituted dopamine and 2-amino-6,7-dihydroxy-1,2,3,4-tetrahydronaphthalene analogues: behavioral effects in lesioned and reserpinized mice"**

(73) Proprietor: **SMITHKLINE BECKMAN CORPORATION**
**P.O. Box 7929 1 Franklin Plaza**
**Philadelphia Pennsylvania 19101 (US)**

(72) Inventor: **Gaitanopoulos, Dimitri**
**1134 Bayless Place**
**Eagleville Pennsylvania 19403 (US)**
Inventor: **Weinstock, Joseph**
**1234 Pothouse Road**
**Phoenixville Pennsylvania 19406 (US)**

(74) Representative: **Waters, David Martin, Dr. et al**
**Smith Kline & French Laboratories Ltd. Patent Department Mundells**
**Welwyn Garden City Hertfordshire AL7 1EY (GB)**

**EP 0 209 275 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue St-Denis, 75001 Paris, France

**Description**

This invention comprises new halodihydroxy benzsubstituted 2-aminotetralins which have potent and selective dopaminergic activity as well as medical methods and compositions using them.

Background of the Invention

6,7-Dihydroxy-2-aminotetralin (ADTN) is known to be a dopaminergic agent with potent stimulating (agonist) activity at both $D_1$ and $D_2$ receptors in the peripheral dopaminergic systems. $D_2$ receptors are located presynaptically. Agonistic activity at $D_2$ sites inhibits the natural release of norepinephrine, G.N. Woodruff, TIPS 1982 59 (1982).

A series of patents by J. Pless, such as Swiss Patents 637 373, 637 364 and, especially, 637 363, describe compounds whose structures have two or three mixed halohydroxy substituents in the tetralin nucleus. The compounds are described to have stimulating activity at α- and β-adrenoceptors as well as at dopamine receptors. In our tests, selected representatives of this series have been demonstrated to have reduced dopaminergic activity and little selectivity over ADTN itself.

Description of the Invention

The compounds of this invention are illustrated by the following structural formula :

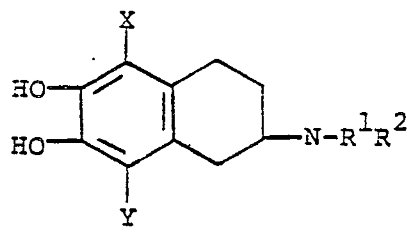

(I)

in which :

$R^1$ and $R^2$ are, each, hydrogen, n-propyl, n-butyl or 4-hydroxyphenethyl ;

X is hydrogen or halo such as fluoro, bromo or chloro ; and

Y is, when X is hydrogen, halo or, when X is halo, hydrogen.

The pharmaceutically acceptable, acid addition salts of the bases of formula I are also included in this invention such as the hydrohalide, sulfate, phosphate, sulfamate, ethanedisulfonate, methanesulfonate or maleate salts.

Also included in this invention are the optical isomers of the compounds of formula I. These are isolated by the methods used in the prior art for resolving ADTN.

One skilled in the art will recognize that the compounds of this invention are the 5 or 8-halo derivatives of ADTN. The Swiss patents, which are mentioned above, describe the preparation of ADTN congeners whose structures have a hydroxy substituent along with one or two halo substituents in the benz-ring of ADTN. Such compounds are less potent then ADTN in the tests described below. On the other hand, the compounds of formula I are very potent dopaminergic compounds and demonstrate selectivity of action at the pre- and post-synaptic sites as well.

The following data in standard protocols to determine receptor binding at the $D_1$ and $D_2$ sites are selected to demonstrate the unexpected potency and selectivity of the dopaminergic compounds of this invention. Other tests have been run on many of these compounds but are not believed relevant to a fair demonstration of the biological properties of the compounds of this invention.

A) $D_2$ Activity — Competition for $^3$H-Spiroperidol in Bovine Anterior Pituitary Tissue.

Test Procedure

The assay was performed using homogenized and washed membrane preparations from bovine anterior pituitary. Incubation proceeded for 20 minutes at 37° in 50 mM Tris-HCl, pH 7.4, containing 10 mM magnesium sulfate, 2 mM ethylenediamine tetra-acetic acid and 0.1 % ascorbate. The membrane-bound radioactivity was trapped by rapid filtration over glass fiber (GF/C) filters. In each experiment, the amount of $^3$H-spiroperidol bound was determined in the absence (total) and presence (non-specific) of $10^{-6}$M (+)-butaclamol, the difference yielding specific $^3$H-spiroperidol binding. The ability of each compound to compete with $^3$H-spiroperidol (approximately .25 nM) was tested at concentrations of $10^{-7}$ and $10^{-5}$. If a compound was found to displace spiroperidol by ≥ 50 % at a concentration of $10^{-5}$ M, it was considered to have significant activity and was further tested to obtain an $IC_{50}$ for competition against spiroperidol. The $K_{Bind}$ ($K_B$) of a compound was calculated from the equation :

$K_B = (IC_{50})/(1 + L/K_D)$ where L is the concentration of $^3H$-spiroperidol and $K_D$ is the equilibrium dissociation constant for spiroperidol (0.3 nM).

B) $D_1$ Activity — Competition for $^3H$-Fenoldopam in Rat Striatum Tissue.

Test Procedure

The assay was performed using homogenized and washed membrane preparations from rat caudate nuclei. Tissues were preincubated for 15 min at 37° in 50 mM Tris-HCl, pH 7.4, 10 mM magnesium sulfate, 2 mM ethylenediamine tetra-acetic acid and 0.1 % ascorbate. The binding assay was then carried out in the above buffer also containing 10 μm pargyline, $^3H$-fenoldopam and test compounds. The membrane-bound radioactivity was trapped by rapid filtration over glass fiber (GF/C) filters. In each experiment the amount of $^3H$-fenoldopam bound was determined in the absence (total) and presence (non-specific) of $10^{-6}$M (+)-butaclamol, the difference yielding specific $^3H$-fenoldopam binding. The ability of each compound to compete with $^3H$-fenoldopam (approximately 2.0 nM) was tested at concentrations of $10^{-7}$ and $10^{-6}$M. If a compound was found to displace fenoldopam by 50 % at a concentration of $10^{-6}$M, it was considered to have significant activity and was further tested to obtain an $IC_{50}$ for competition against fenoldopam. The $K_{Bind}$ ($K_B$) of a compound was calculated from the equation: $K_B = (IC_{50})(1 + L/K_D)$ where L is the concentration of $^3H$-fenoldopam and $K_D$ is the equilibrium dissociation constant for fenoldopam (2.0 nM).

Table I

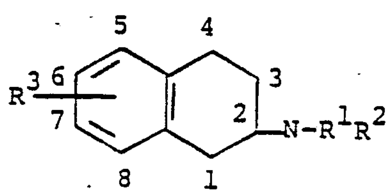

| | $R^3$ | $R^1$ | $R^2$ | $D_1$* (μM) | | $D_2$ (μM) | |
|---|---|---|---|---|---|---|---|
| A. | 8-Cl; 6,7-$(OH)_2$ | n-Pr | HO-Ph-Et | 0.27 | (3) | 0.033 | (2) |
| B. | 8-Cl; 6,7-$(OH)_2$ | H | HO-Ph-Et | 1.45 | (2) | 0.405 | (2) |
| C. | 8-Cl; 6,7-$(OH)_2$ | n-Pr | n-Pr | 0.62 | (3) | 0.24 | (3) |
| D. | 5-F; 6,7-$(OH)_2$ | H | H | 0.072 | (2) | 0.52 | (3) |
| E. | 8-F; 6,7-$(OH)_2$ | H | H | 0.062 | (2) | 1.27 | (3) |
| F. | 5-Cl; 6,7-$(OH)_2$ | H | H | 0.64 | (3) | 0.47 | (3) |
| G. | 8-Cl; 6,7-$(OH)_2$ | H | H | 0.043 | (3) | 3.16 | (3). |

* number of tests.

The potency and selectivity of representative compounds of this invention are demonstrated by these data. Compounds A, B and C, which are N-substituted, have $D_2$ to $D_1$ ratios of from about 2 to 1 about 10 to 1. ADTN has a reverse ratio of 1 to 3 (see Compound H of Table II below). It is known to the art that selective $D_2$ activity is desirable for treating abnormal conditions by means of the dopamine receptor system (U.S. 4,465,692).

On the other hand, the primary amine series of this invention, for example D → G, show a greater $D_1$ selectivity than does ADTN ; compound D, 1 to 7 ; E, 1 to 27 ; and G, 1 to 80. In certain cardiovascular conditions, this may be of advantage.

The data in Table II were derived from testing the prior art compounds. The same headings as in Table I are used.

Table II

| | | | | | | | |
|---|---|---|---|---|---|---|---|
| H. | 6,7-$(OH)_2$ | H | H | 0.07 | | 0.21 | |
| I. | 6,8-$(Cl)_2$; 7-OH | n-Pr | n-Pr | >10 | (2) | 10.4 | (3) |
| J. | 6,7-$(Cl)_2$; 7-OH | H | H | 2.97 | (2) | 30 | (4) |
| K. | 6-Cl; 7-OH | H | H | 1.44 | (2) | 1.05 | (2) |

Tabelle 2 (Fortsetzung)

| | | | | | |
|---|---|---|---|---|---|
| L. | 6-F; 7-OH | H | H | 0.37 (2) | 0.66 (3) |
| M. | 6-Cl; 7-OH | n-Pr | n-Pr | 4.71 (2) | 1.63 (3) |
| N. | 7-Cl; 6-OH | H | H | 1.97 (3) | 4.47 (4) |
| O. | 5-Cl; 6-OH | H | H | >10 (2) | 11.6 (3) |
| P. | Dopamine | | | 0.15 | 2.35 |

These data, combined with those in Table I, demonstrate that random benz-halogenation gives ADTN congeners which have low potency. N-alkylation (see Compound I) almost destroys activity in the prior art compounds.

In addition to the data in Table I, Compound G demonstrated potent natriuretic activity in the spontaneous hypertensive rat protocol at a hypotensive dose (3.12 mg/kg i.p.) while ADTN was inactive as a natriuretic at hypotensive doses. Compounds D, E, F, G and H of Tables I and II have also demonstrated $\alpha_2$ activity in the clonidine binding protocol. In the standard rabbit ear artery protocol for pre-junctional dopaminergic activity, the fluoro congeners also demonstrated very potent activity compared with their chloro counterparts : Compound D, $ED_{50}$ of 2.14 nM ; Compound E, $EC_{50}$ of 5.0 nM ; Compound F, $EC_{50}$ of 50 nM ; and Compound G, $EC_{50}$ of 513 nM.

The compounds of this invention are prepared by the following reaction sequences :

(A)

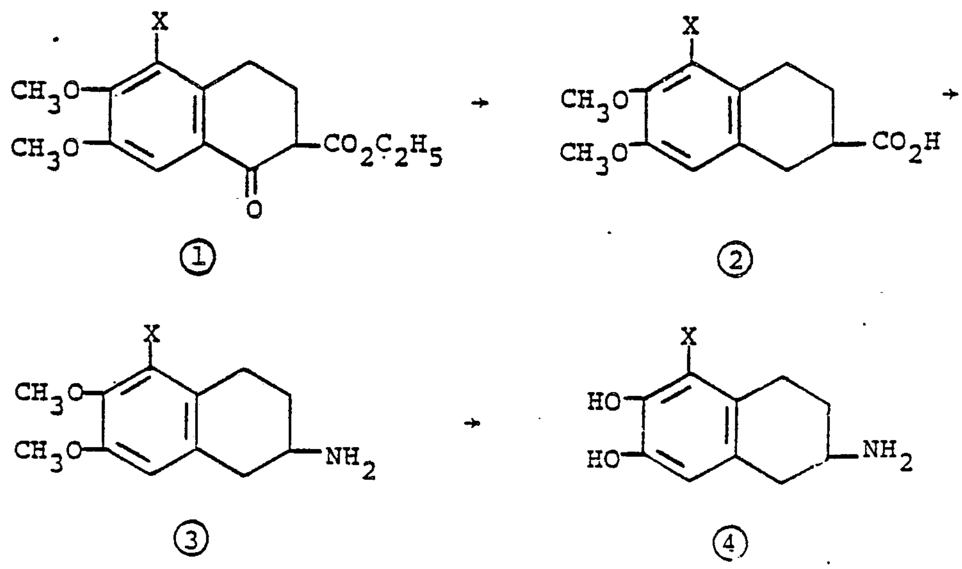

① ② ③ ④

(B)

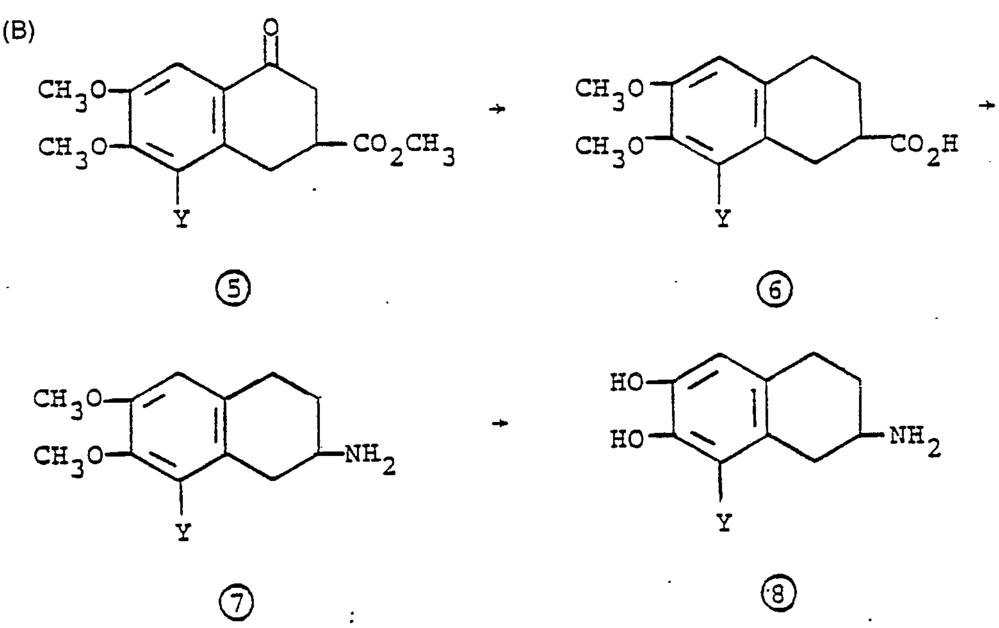

⑤ ⑥ ⑦ ⑧

(C)

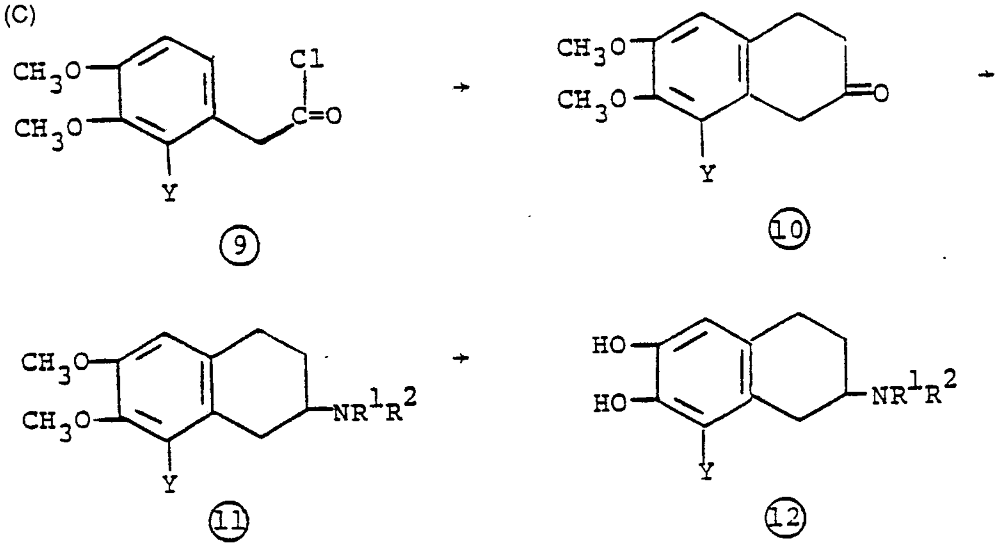

9 10 11 12

Each of the reaction sequences, which may be used by one skilled in the art to prepare the compounds of this invention and are outlined above, involve the insertion of a 2-amino function into a tetralin which is trisubstituted in the benz-ring. The amino function may be generated by a Curtius rearrangement of a 2-carboxy functional group (A or B) or by reacting a 2-keto group with an amine (C).

In each of the sequences, the primary amine, Compound 3, 7 or 11, whose 6,7-dihydroxy system is protected, is optionally N-alkylated to prepare the preferred N-alkylated compounds of formula I. The alkylation usually proceeds stepwise by means of a reductive alkylation using an aldehyde in a reducing milieu or using reaction with an acyl halide to form an amide which is then reduced, for example by borane. The protective groups are removed using any ether splitting agent such as hydrobromic acid, hydriodic acid, boron tribromide or boron trichloride.

Details of these sequences and preparation of starting materials are present in the illustrative examples below.

The compounds of this invention have utility, as specific dopamine agonists, in the treatment of disorders of the cardiovascular system, especially to treat hypertension, to treat angina pectoris, to treat the symptoms of congestive heart failure or to improve kidney blood flow.

The pharmaceutical compositions of this invention, which have pharmacodynamic activity within the cardiovascular system, are prepared in conventional dosage unit forms by incorporating a compound of formula I, or a pharmaceutically acceptable isomer or acid addition salt thereof, into a nontoxic pharmaceutical carrier according to accepted pharmacy procedures in a nontoxic quantity sufficient to produce the desired pharmacodynamic activity in an animal or human patient. Preferably, the compositions will contain the active ingredient in an active but nontoxic quantity which is selected from the range of about 25 mg to about 300 mg, preferably about 100-250 mg of active ingredient, as the base, per dosage unit. This quantity is selected by considering the specific biological activity desired, the route of administration, that is, whether oral or parenteral, and the condition and size of the patient.

The pharmaceutical carrier employed for the dosage units is, for example, either a solid or liquid. Exemplary of solid carriers are lactose, terra alba, sucrose, talc, gelatin, agar, pectin, acacia, magnesium stearate or stearic acid. Exemplary of liquid carriers are isotonic saline for parenteral use or syrup, peanut oil, olive oil or water for soft gelatin capsules. Similarly, the carrier or diluent may include any time delay material well known to the art, such as cellulose esters or ethers and glyceryl esters alone or admixed with a wax.

A wide variety of pharmaceutical forms can be employed. Thus, if a solid carrier for oral, trans-dermal or rectal administration is used, the mixed preparation can be tableted, placed in a hard gelatin capsule in powder or sustained release pellet form, dermal patch, in a suppository or in the form of a troche or lozenge. The amount of solid carrier will vary widely but, preferably, will be from about 25 mg to about 1 g per dose. If a liquid carrier is used, the preparation will be in the form of a syrup, emulsion, soft gelatin capsule, sterile injection liquid for an ampul or multidose vial, an aqueous or nonaqueous liquid suspension for oral administration, or a trans-dermal device.

Advantageously, doses selected from the dosage unit ranges given above will be administered several times, such as from one to five times, a day. The daily dosage regimen is selected from the range of about 100 mg to about 1.0 g, preferably 100-500 mg for oral administration and 25-250 mg for parenteral administration. When the method described above is carried out, selective dopaminergic activity is produced.

The following examples are designed solely to illustrate the preparation and use of the compounds of this invention. The temperatures herein are Centigrade. Other variations of these examples will be obvious to those skilled in the art.

Example 1

2-Fluoro-3,4-dimethoxyphenylacetic Acid

A mixture of 50.42 g (0.258 mol) of 3,4-dimethoxy-2-fluorophenylacetonitrile, potassium hydroxide (254.7 g, 4,54 mol), 500 ml of ethanol and 500 ml of water was stirred and refluxed for 20 hours. The resulting solution was concentrated to a volume of about 500 ml. After being cooled to 25°, the solution was extracted twice with ether. The aqueous phase was filtered, and the filtrate was poured slowly into a stirred solution of 450 ml of concentrated hydrochloric acid in 600 ml of water. The resulting suspension was stirred for 1 hour at — 10° to 0°, and, then, filtered. After the solid was washed with water, it was dried to give 44.0 g (80 %) of colorless crystals, mp 100-101°.

2-Fluoro-3,4-dimethoxyphenylethanol

To a stirred solution of 52.4 g (0.245 mol) of 3,4-dimethoxy-2-fluorophenylacetic acid in 500 ml of tetrahydrofuran, under argon, was added, dropwise over 1 hour, 295 ml (0.295 mol) of a 1 M solution of borane in tetrahydrofuran. After being stirred at 25° for 24 hours, the reaction mixture was cooled to 0-5° and 160 ml of methanol was added dropwise. The resulting solution was concentrated in vacuo to leave a liquid residue. A solution of this residue in 300 ml of ether was washed twice with a saturated solution of sodium bicarbonate. The ethereal solution was dried, concentrated and the residue was distilled to give 46.63 g (98 %) of a colorless liquid, bp 128-130° (20 Pa ; 0.15 torr).

2-(2-Fluoro-3,4-dimethoxyphenyl) ethyl Tosylate

Tosyl chloride (8.09 g, 0.042 mol) was added in small portions to a stirred solution of the alcohol (4.2 g, 0.021 mol) in 15 ml of dry pyridine. The resulting suspension was stirred at 5° for 16 hours and then 5 ml of water was added slowly. After the mixture was stirred at 5° for 1 hour, it was diluted with 50 ml of cold water. The mixture was extracted with ether, and the ethereal extracts were washed with 0.1 N HCl, water, and finally with a saturated solution of sodium bicarbonate. The ether solution was dried and concentrated to leave a 6.64 g (89 %) of a solid residue ; mp 72-73° after trituration with petroleum ether.

Diethyl α-[2-(2-Fluoro-3,4-dimethoxyphenyl)-ethyl] malonate

Diethyl malonate (13.73 g 85.7 mmol) was added dropwise at 20° to a stirred suspension of 1.38 g (55.8 mmol) of sodium hydride in 55 ml of tetrahydrofuran under an atmosphere of argon. To the resulting reaction mixture was added, dropwise, a solution of 15.19 g (42.9 mmol) of the tosylate. The mixture was refluxed for 40 hours under argon. It was concentrated in vacuo. The residue was partitioned between 50 ml of ether and 50 ml of water. The layers were separated and the aqueous phase was extracted with ether. After the combined ether extracts were washed with water and brine, they were dried and concentrated. The residue was fractionally distilled to give 9.80 g (67 %) of a colorless liquid, bp 175° (17.3 Pa : 0.13 torr).

Ethyl hydrogen α-[2-(2-Fluoro-3,4-dimethoxyphenyl)-ethyl] malonate

A solution of 1.57 g (28 mmol) of potassium hydroxide in ethanol was added dropwise, over a period of 1 hour, to a stirred solution of 9.59 g (28 mmol) of the di-ester in 25 ml of ethanol. After being stirred at ambient temperature for 60 hours, the mixture was concentrated in vacuo. The residue was extracted with ether/water, 1 : 1. The aqueous phase was acidified with 2.5 N hydrochloric acid and the mixture was extracted with ether. After being washed with water and brine, the ether extract was dried and concentrated in vacuo to give 7.32 g (83 %) of a colorless syrup.

Ethyl 5-fluoro-6,7-dimethoxy-1-oxo-1,2,3,4-tetrahydro-2-naphthoate

A solution of 6.67 g (21.2 mmol) of the half ester in 35 ml of approximately 3 M polyphosphate ester in chloroform was stirred under argon at ambient temperature for 24 hours. The reaction mixture was poured into 50 g of crushed ice with vigorous stirring. After the layers were separated, the aqueous phase was extracted with ether. The combined organic extracts were washed, successively, with water, 5 % aqueous sodium bicarbonate, water and brine. After being dried (anhydrous sodium sulfate), the organic extract was concentrated to give 3.05 g of a viscous liquid that solidified on standing at 25°. Uncyclized malonate (2.56 g) was recovered from the sodium bicarbonate extracts. This was treated with polyphosphate ester as described above to give an additional 1.88 g of crude product. The combined crude product (4.93 g) was chromatographed by medium pressure liquid chromatography usind a 2.5 × 50 cm column packed with 124 g of 230-400 mesh silica gel and a mobile phase of 3.33 ml of 97 % formic acid per liter of chloroform. Fifty-four fractions were collected at a flow rate of 5 ml/min. Fractions 6-34 were combined and concentrated to give 1.75 g of a viscous liquid that crystallized on standing at 25° ; TLC

(silica gel GF, 100 : 0.2-$CHCl_3$/HCOOH) showed one spot at $R_f$ = 0.35. Recrystalization from ethanol gave 1.23 g (20 %) of colorless needles, mp 77-79°.

5-Fluoro-6,7-dimethoxy-1,2,3,4-tetrahydro-2-naphthoic Acid

A mixture of 1.86 g (6.28 mmol) of the above compound, 0.39 g of 10 % palladium-on-carbon, 0.39 ml of 70 % perchloric acid and 25 ml of acetic acid was hydrogenated for 4 hours on a Parr apparatus at 25° and a moderate hydrogen pressure. Sodium acetate (0.39 g) was added and the mixture was stirred for 5 minutes. The mixture was filtered. The filtrate was concentrated in vacuo. The residue was taken into ether. After the ethereal solution was washed with a saturated solution of sodium bicarbonate, it was dried and concentrated to give 1.70 g (96 %) of crude ethyl 5-fluoro-6,7-dimethoxy-1,2,3,4-tetrahydro-2-naphthoate. To this ester (1.52 g, 5.38 mmol) in 10 ml of methanol was added a solution of 0.6 g (10.8 mmol) of potassium hydroxide in 10 ml of water. The resulting mixture was stirred and refluxed for 2 hours. After the methanol was distilled from the reaction mixture, it was diluted with 50 ml of water. The resulting solution was washed with ether and then it was acidified with 2.5 N hydrochloric acid to give 1.25 g (93 %) of crystalline product, mp 178-181°.

Example 2

2-(2-Chloro-3,4-dimethoxyphenyl) ethanol

This compound was prepared by borane reduction of 2-chloro-3,4-dimethoxyphenylacetic acid in the same manner as described for the fluoro analogue of Example 1. The yield of colorless liquid, bp 128-132° (0.2 torr), was 94 %.

2-(2-Chloro-3,4-dimethoxyphenyl) ethyl bromide

Phosphorus tribromide (3.77 g, 13.9 mmol) in 30 ml of ether was added to a stirred solution of 6.03 g (27.8 mmol) of the ethanol in 30 ml of ether. The solution was refluxed for 1 hour, cooled to 5°, and, then, cautiously poured into 60 g of crushed ice. After the ether phase was separated and washed successively with a saturated solution of sodium bicarbonate, water and brine, it was dried and concentrated. The residual liquid was distilled and the fraction (5.30 g, 68 %), bp 112-115° (20 Pa ; 0.15 torr), was collected.

Diethyl 2-[2-(2-Chloro-3,4-dimethoxyphenyl)-ethyl] malonate

This compound was prepared from 2-(2-chloro-3,4-dimethoxyphenyl) ethyl bromide and diethyl malonate by essentially the same procedure described above (48 hour reflux period). The product (83 %) was obtained as a colorless liquid, bp 172° (20 Pa ; 0.15 torr).

Ethyl hydrogen α-[2-(2-Chloro-3,4-dimethoxyphenyl)-ethyl] malonate

This compound was prepared from the bromide by same procedure described in Example 1. The product was obtained as a viscous, colorless liquid ; TLC (silica gel, GF, 95 : 5 : 0.2 $CHCl_3$/$CH_3OH$/HCOOH) gave a single spot, Rf 0.9. It was employed for further reaction without additional purification.

Ethyl 5-chloro-6,7-dimethoxy-1-oxo-1,2,3,4-tetrahydro-2-naphthoate

This compound was prepared from the half ester in the same manner as described in Example 1. In this case, however, the crude product was purified by silica gel column chromatography using chloroform for elution. All fractions have a single spot at $R_f$ 0.3 (silica gel GF, $CHCl_3$) were combined and concentrated. The residue was crystallized from aqueous ethanol to give colorless crystals (41 % yield), mp 79-83°.

5-Chloro-6,7-dimethoxy-1,2,3,4-tetrahydro-2-naphthoic acid

This compound was prepared from the bicyclic compound in the same fashion as that described for conversion in Example 1. Crude intermediate ethyl 5-chloro-6,7-dimethoxy-1,2,3,4-tetrahydro-2-naphthoate (96 % yield) was obtained as colorless crystals, mp 56-57°. The acid (97 % yield) was a colorless solid, mp 172-174°.

Example 3

2-Fluoro-3,4-dimethoxyphenylacetyl chloride

A stirred suspension of 43.2 g (0.2 mol) of the titled acid and 71.8 g (0.6 mol) of thionyl chloride in 460 ml of toluene was heated at 100° for 2.5 hours. Stirring was continued at 25° for 16 hours and then the solution was concentrated under reduced pressure. Distillation of the residue afforded 45.65 g (97 %) of a pale pink liquid, bp 128-132° (34.6 Pa ; 0.26 torr).

8-Fluoro-6,7-dimethoxy-2-tetralone

A solution of 45.5 g (0.196 mol) of the acyl chloride in 200 ml of chloroform was added dropwise during 1.5 hours to a stirred suspension of aluminum chloride (52.2 g, 0.392 mol) in 800 ml of methylene chloride at —78°. Ethylene was bubbled rapidly through the mixture for 25 minutes. After removal of the cooling bath, stirring was continued for 3.5 hours and 250 ml of water was added dropwise with caution. The organic layer was separated and washed with 2 N hydrochloric acid, a saturated solution of sodium bicarbonate and brine. After being dried, the extract was concentrated in vacuo to give 44.05 g of a viscous liquid which was triturated with a small volume of cold ether to give 11.3 g of a crude monohydroxy-monomethoxy derivative.

A mixture of 10.28 g (48.9 mmol) of this crude material, 7.4 g (58.7 mmol) of dimethyl sulfate and 31.3 g (0.227 mol) of potassium carbonate in 80 ml of dimethylformamide was stirred at 100° for 1.5 hours. The cooled mixture was diluted with water and extracted with ether. The ether extracts were washed with water, dried and concentrated. The residue was chromatographed by MPLC (silica gel 60, 230-240 mesh ; mobile phase 98 : 2, $CH_2Cl_2/CH_3OH$). Fractions giving a single spot at $R_f$ 0.3 (70 : 30 $C_6H_{14}$—$Et_2O$) were combined, concentrated and the residual solid was recrystallized from cyclohexane-n-hexane to give 2.12 g (19 %) of colorless crystals, mp 94-95°.

2-Benzylamino-8-fluoro-6,7-dimethoxytetralin hydrochloride

A solution of 2.03 g (9 mmol) of the ketone, p-toluene sulfonic acid (0.06 g) and benzylamine (1.16 g, 11 mmol) in 100 ml of toluene was refluxed azeotropically under argon for 2 hours. After carbon dioxide was bubbled through the solution, the latter was washed successively with water, a saturated aqueous sodium bicarbonate solution and brine, dried, and concentrated. A mixture of the residual imine, 280 mg of platinum oxide and 100 ml of ethanol was hydrogenated at 25° and medium pressure hydrogen for 1.5 hours. After the mixture was filtered, the filtrate was made acidic with hydrochloric acid and concentrated in vacuo. The residual solid was recrystallized from methanol-ethanol to give 2.12 g (70 %) of a white solid, mp 247-250°.

2-Amino-8-fluoro-6,7-dimethoxytetralin hydrochloride

A suspension of 2.08 g (5.9 mmol) of the benzylamine, 0.5 ml of 11 N hydrochloric acid and 100 ml of ethanol was hydrogenated over palladium catalyst at 60° and medium hydrogen pressure for 5 hours. The mixture was filtered and the filtrate concentrated. Recrystallization of the solid residue from methanol-ethyl acetate gave 1.45 g (94 %) of colorless crystals, mp 225-228° (dec).

## Example 4

Methyl 8-chloro-6,7-dimethoxy-4-oxo-1,2,3,4-tetrahydro-2-naphthoate

A warm solution of 2-chloro-veratraldehyde (100.3 g, 0.5 mol) and dimethyl succinate (83.1 g, 0.57 mol) in 170 ml of methanol was added to a refluxing solution of sodium methoxide in methanol, prepared by the cautious portionwise addition of 13.8 g (0.6 g-atom) of sodium metal to 500 ml of methanol under nitrogen. After the mixture was refluxed for 2 hours, 350 ml of solvent was distilled off. The remaining solution was cooled to 10°, made acidic with 2 N hydrochloric acid and diluted with 1.5 l of water. Precipitated solid was filtered and partitioned between equal volumes of ether and a saturated aqueous solution of sodium bicarbonate. The aqueous solution was acidified with concentrated hydrochloric acid. After the resulting mixture was extracted with ethyl acetate, the organic extract was washed (water), dried, and concentrated to give 52.8 g of a solid benzylidene derivative, mp 140-143° after recrystallization from ethyl acetate-hexane. This was dissolved in ethyl acetate and hydrogenated in the presence of 10 % palladium-on-carbon at 25° and medium pressure to give 52.1 g of a benzyl derivative, mp 118-121°. A mixture of this material and 375 ml of methane sulfonic acid was heated at 100° for 15 min. The resulting solution was poured onto 2 l of ice-water. Precipitated solid was filtered, washed with water, dried in vacuo at 80° and recrystallized from aqueous methanol to give 43.1 g (92 %) of crystalline solid, mp 146-147°.

Methyl 8-chloro-6,7-dimethoxy-1,2,3,4-tetrahydro-2-naphthoate

This compound was prepared by reductive hydrogenolysis of the oxo compound in the same manner as described above. The crystals, mp 92-93° (99 % yield), were recrystallized from methanol ; TLC (silica

gel GF, chloroform) showed a single spot at $R_f$ 0.4.

8-Chloro-6,7-dimethoxy-1,2,3,4-tetrahydro-2-naphthoic acid

A suspension of 29.6 g (97 mmol) of the ester in a solution of 10.9 g (0.184 mol) of potassium hydrate in 200 ml of 50 % aqueous methanol was stirred and refluxed for 24 hours. The solution was concentrated to 100 ml in vacuo, cooled, washed with ether and made acidic with concentrated hydrochloric acid to give 25.26 g of colorless crystals, mp 212-214°, after recrystallization from ethyl acetate.

2-Amino-8-chloro-6,7-dimethoxytetralin hydrochloride

This compound was prepared from the acid by Method B of Example 7. The yield of colorless crystals, mp 269-271 (dec.), after trituration with ether was 96 %.

## Example 5

8-Chloro-6,7-dimethoxy-2-di(n-propyl) aminotetralin hydrochloride

A mixture of the amine from Example 4 (5.56 g, 20 mmol), 5.81 g (0.1 mol) of propionaldehyde, 1.64 g (20 mmol) of sodium acetate and 2 g of 10 % palladium-on-carbon was hydrogenated for 10 hours at 25° and with medium hydrogen pressure. The mixture was filtered and the filtrate was concentrated in vacuo. The residue was suspended in water, the mixture was made alkaline with 10 N sodium hydroxide and extracted with ether. After the ethereal solution had been washed (water) and dried, it was concentrated. The residue was taken into ether and acidified with gaseous hydrogen chloride. The resulting hydrochloride was recrystallized from acetonitrile-ether to give 3.77 g (52 %) of crystals, mp 158-160°.

## Example 6

8-Chloro-6,7-dimethoxy-2-(4-methoxyphenylacetyl)-aminotetralin

To a suspension of 2-amino-8-chloro-6,7-dimethoxy-tetralin (2.5 g, 9 mmol) in 50 ml of tetrahydrofuran was added 2.17 g (21.4 mmol) of triethylamine. The mixture was stirred for 10 minutes at ambient temperature, then it was cooled to 0° and 2.2 g (12 mmol) of 4-methoxy-phenylacetyl chloride in 10 ml of tetrahydrofuran was added dropwise. The mixture was stirred at 25° for 16 hours. Then, it was filtered. The filtrate was concentrated in vacuo. A solution of the residual solid in chloroform was washed (water, saturated sodium bicarbonate solution, water), dried and concentrated. The residue was triturated with a small volume of cold ether to give 2.35 g (67 %) of colorless crystals, mp 144-147°.

8-Chloro-6,7-dimethoxy-2-[2-(4-methoxyphenyl)-ethylamino] tetralin hydrochloride

A solution of 2.31 g (5.92 mmol) of the amide in 20 ml of tetrahydrofuran was added dropwise at 0° to a stirred solution of 14 ml of a 1 M solution of borane in tetrahydrofuran under argon. After the reaction mixture had been refluxed for 2 hours, it was cooled, 4 ml of 3 N hydrochloric acid was added and the borane was removed by distillation. The resulting suspension was cooled to 10°, 30 ml of tetrahydrofuran was added, the crystals (2.12 g, 87 %), mp 268-270°, were filtered off.

8-Chloro-6,7-dimethoxy-2-[N-[2-(4-methoxyphenyl)-ethyl]-N-n-propylamino] tetralin

To a stirred suspension of the secondary amine (1.67 g, 4.05 mmol) and potassium carbonate (3.0 g, 21.7 mmol) in 10 ml of water and 10 ml of chloroform was added 0.93 g (10.1 mmol) of propionyl chloride. After being stirred at 25° for 1 hour, the mixture was filtered and the organic phase was separated. The chloroform solution was washed (acid/water), dried and concentrated to give 1.48 g of a viscous amide. This amide was reduced with borane as described above. An aqueous solution of the resulting crude hydrochloride was made alkaline with 2.5 N sodium hydroxide. After the mixture had been extracted with ether, the extracts were washed (water), dried and concentrated to give 1.35 g (96 %) of a crude product ; TLC (silica gel GF (98 : 2 — $CHCl_3$ : $CH_3OH$) $R_f$ = 0.45. This material was used for demethylation without further purification.

## Example 7

### General Methods for Preparing 6,7-Dimethoxy-2-aminotetralins

Method A

The appropriate carboxylic acid was converted to the corresponding isocyanate by the mixed

carboxylic-carbonic anhydride modification of the Curtius reaction as described by Weinstock, J. Org. Chem. 26 3511 (1968). The crude isocyanate was dissolved in benzyl alcohol (5 ml per g of isocyanate). The solution was heated at 100° for 6 hours. Excess benzyl alcohol was removed in vacuo to leave a residual dimethoxylated 2-carbobenzoxamidotetralin, some of which crystallized upon trituration with n-hexane. Hydrogenolysis of the benzyl carbonate to the methoxylated amine and conversion to a salt was carried out according to the general procedure of Nichols et al. J. Med. Chem. 17 161 (1974).

Prepared by. Method A was 2-amino-5-fluoro-6,7-dimethoxytetralin hydrochloride ; mp 237-239°, from methanol-ether, 99 % yield.

Anal. Calcd. for $C_{12}H_{16}FNO_2 \cdot HCl$ : C, 54.60 ; H, 6.59 ; N, 5.31. Found : C, 54.30 ; H, 6.43 ; N, 5.39.

Method B

The appropriate tetralin-2-carboxylic acid (0.1 mol) was stirred and heated with 50 ml of thionyl chloride at 65° for 1 hour. Resulting acid chloride was converted to an isocyanate by the wet sodium azide procedure, Kaiser et al. J. Med. Pharm. Chem. 5 1243 (1962). Isocyanate was converted to a benzyl carbonate as described in Method A above. Trituration of a carbamate with a solvent such as 2-propanol gave crystals. The carbamate was converted to amine as described in Method A.

Prepared by this method was 2-amino-5-chloro-6,7-dimethoxytetralin hydrochloride ; mp 242-244°, from methanol-ethyl acetate, 48 % yield.

Anal. Calcd. for $C_{12}H_{16}ClNO_2 \cdot HCl$ : C, 51.81 ; H, 6.16 ; N, 5.04. Found : C, 51.42 ; H, 6.13 ; 4.83.

Example 8

General Method for N-Alkylating 6,7-dimethoxy-2-aminotetralins

A 6,7-dimethoxy-2-aminotetralin (20 mmol) was heated with 20 ml of propionic anhydride at 100° for 2 hours. The solution was poured into water and the mixture was extracted with methylene chloride. The organic extract was washed (5 % aqueous sodium bicarbonate, water, brine), dried and concentrated. To a stirred solution of the residue N-propionamide in 2 ml of tetrahydrofuran under nitrogen was added 30 ml of 1 M borane in tetrahydrofuran. The stirred mixture was refluxed for 4 hours, cooled, and 2.5 N hydrochloride acid was added until the evolution of gas stopped. The mixture was heated at 100°, allowing the tetrahydrofuran to evaporate. The resulting solid was collected. This hydrochloride was converted to the base which was again propionylated and reduced in the same manner to give an N,N-dipropyl derivative. In most instances, the resulting aqueous solution of hydrochloride was converted to the base by addition of 2 N sodium hydroxide. The base was extracted with ethyl acetate. The extracts were washed with water, dried and concentrated to afford residual tertiary amine that was converted into a hydrochloride in an organic solvent with hydrogen chloride gas.

Example 9

General Methods for Demethylation

Method A

A 0.2 M solution of boron tribromide in methylene chloride (100 ml) was added dropwise to a stirred mixture of 20 mmol of the 6,7-dimethoxy-2-aminotetralin in 100 ml of methylene chloride at —78°. The cooling bath was removed and the reaction was stirred at ambient temperature for 24 hours. After the mixture was cooled in an ice-water bath, 100 ml of methanol was added dropwise. The resulting solution was concentrated. The residue was dissolved in 100 ml of methanol and the solution was again concentrated. This procedure was repeated three times, and then the solid residue was recrystallized from methanol-ethyl acetate to give the amine hydrobromide.

Method B

A stirred solution of 10 mmol of the 6,7-dimethoxy-2-aminotetralin and 30 ml of freshly distilled concentrated hydrobromic acid was heated at 110° for 2.5 hours. The solution was concentrated in vacuo to about 10-20 ml. It was cooled to 0-10°. The crystalline hydrobromide was filtered off and recrystallized. In some cases the hydrobromide, treated with base such as sodium carbonate, was converted to the free base, chromatographed, and then treated with hydrogen chloride to give a hydrochloride.

The following compounds are prepared by the methods described :

(1) 5-Fluoro-6,7-dihydroxy-2-aminotetralin as the hydrobromide ; mp 269-270° (dec.), 94 % using method B.

Anal. Calcd. for $C_{10}H_{12}FNO_2HBr$ : C, 43.18 ; H, 4.71 ; N, 5.04. Found : C, 43.56 ; H, 4.68 ; N, 5.18.

(2) 5-Chloro-6,7-dihydroxy-2-aminotetralin as the hydrobromide ; mp 250° (dec.), 98 % using method B.

Anal. Calcd. for $C_{10}H_{12}ClNO_2HBr$ : C, 40.77 ; H, 4.45 ; N, 4.76. Found : C, 41.13 ; H, 4.60 ; N, 4.70.

(3) 8-Fluoro-6,7-dihydroxy-2-aminotetralin as the hydrobromide ; mp 225-229°, 75 % using method B.

Anal. Calcd. for $C_{10}H_{12}FNO_2 \cdot HBr$ : C, 43.18 ; H, 4.71 ; N, 5.04. Found : C, 43.03 ; H, 4.74 ; N, 4.98.

(4) 8-Chloro-6,7-dihydroxy-2-aminotetralin as the hydrobromide using method B ; mp 253-256° (dec.), 90 % using method B.

Anal. Calcd. for $C_{10}H_{12}ClNO_2 \cdot HBr$ : C, 40.77 ; N, 4.45 ; N, 4.76. Found : C, 40.93 ; H, 4.50 ; N, 4.82.

(5) 8-Chloro-6,7-dihydroxy-2-di-n-propyl aminotetralin as the hydrobromide ; mp 190-191°, 96 % using method B.

Anal. Calcd. for $C_{16}H_{24}ClNO_2 \cdot HBr\ 0.75\ H_20$ ; C, 48.99 ; H, 6.81 ; N, 3.57. Found : C, 49.03 ; H, 6.85 ; N, 3.59.

(6) 8-Chloro-6,7-dihydroxy-2-(4-hydroxyphenethylamino)tetralin as the hydrobromide ; mp 240-241° (dec.), 100 % using method B.

Anal. Calcd. for $C_{18}H_{20}ClNO_3 \cdot HBr$ : C, 52.13 ; H, 5.20 ; N, 3.38. Found : C, 52.44 ; H, 5.35 ; N, 3.53.

(7) 8-Chloro-6,7-dihydroxy-2-[N-(n-propyl)-N-(4-hydroxyphenethyl)-amino] tetralin as the hydrobromide ; mp 195-220° ; 50 % using method B.

Anal. Calcd. for $C_{21}H_{26}ClNO_2HBr \cdot 0.5\ H_2O$ : C, 54.15 ; H, 606 ; N, 3.01. Found : C, 54.32 ; H, 6.02 ; N, 3.09.

(8) 8-Bromo-6,7-dihydroxy-2-(n-propyl-n-butyl-amino)tetralin hydrochloride.

(9) 5-Fluoro-6,7-dihydroxy-2-[N-(n-propyl)-N-(4-hydroxyphenethyl)-amino] tetralin methane sulfonate.

Example 10

8-Chloro-6,7-dihydroxy-2-[N-(n-propyl)-N-(4-hydroxyphenethyl)-amino] tetralin hydrobromide (150 mg) is mixed with 100 mg of lactose and 2 mg of magnesium stearate, filled into a hard gelatin capsule which is administered orally to a patient in need of $D_2$-dopaminergic activity from 1-4 times daily.

Example 11

8-Chloro-6,7-dihydroxy-2-aminotetralin hydrobromide (250 mg) is mixed with 100 mg of lactose and 2 mg of magnesium stearate, filled into a hard gelatin capsule and administered orally to the patient in need of natriuretic or antihypertensive activity from 1-3 times daily.

**Claims** (for the Contracting States : BE, CH, DE, FR, GB, IT, LI, LU, NL, SE)

1. A chemical compound of the formula :

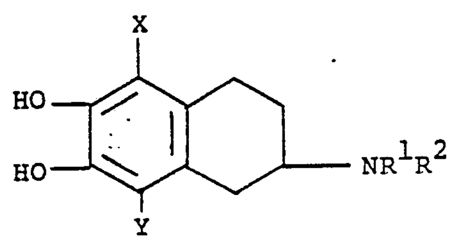

in which :

$R^1$ and $R^2$ are, each, hydrogen, n-propyl, n-butyl or 4-hydroxyphenethyl ;

X is hydrogen or halo ; and

Y is, when X is hydrogen, halo or, when X is halo, hydrogen, or a pharmaceutically acceptable, acid addition salt thereof.

2. A compound according to claim 1 wherein X and Y are chosen from hydrogen, chlorine and fluorine.

3. A compound according to claim 2 wherein $R^1$ and $R^2$ are both hydrogen.

4. A compound according to claim 3 wherein X and Y are chosen from hydrogen and fluorine.

5. A compound according to claim 2 wherein one or both $R^1$ and $R^2$ are other than hydrogen.

6. A compound according to claim 1 which is : 8-chloro-6,7-dihydroxy-2-aminotetralin or a pharmaceutically acceptable acid addition salt thereof.

7. A compound according to claim 1 which is 8-chloro-6,7-dihydroxy-2-[N-(n-propyl)-N-(4-hydroxyphenethyl)amino]tetralin or a pharmaceutically acceptable acid addition salt thereof.

8. A compound according to claim 1 which is chosen from the group comprising :

8-fluoro-6,7-dihydroxy-2-[N-(n-propyl)-N-(4-hydroxyphenethyl)amino]tetralin ;

8-fluoro-6,7-dihydroxy-2-aminotetralin ;

8-chloro-6,7-dihydroxy-2-[N-(hydroxyphenethyl)amino]-tetralin ;

5-chloro-6,7-dihydroxy-2-aminotetralin ;
5-fluoro-6,7-dihydroxy-2-aminotetralin ;
and pharmaceutically acceptable acid addition salts thereof.

9. A pharmaceutical composition comprising a compound as defined in any one of claims 1 to 8 and a pharmaceutically acceptable carrier therefor.

10. A compound according to any one of claims 1 to 8 for use as a dopaminergic agent.

11. A process for the preparation of a compound as defined in any one of claims 1-8, which process comprises the reaction of a compound of the formula (II) :

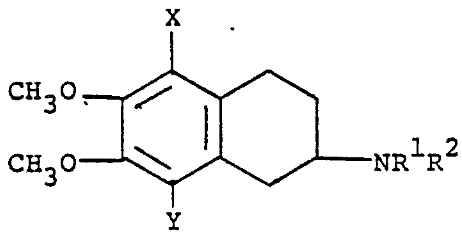

(II)

wherein X, Y, $R^1$ and $R^2$ are as defined in claim 1, with an ether splitting agent.

**Claims** (for the Contracting State AT)

1. A process for the preparation of a compound of the formula :

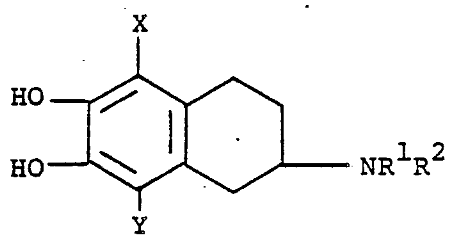

in which :
$R^1$ and $R^2$ are, each, hydrogen, n-propyl, n-butyl or 4-hydroxyphenethyl ;
X is hydrogen or halo ; and
Y is, when X is hydrogen, halo or, when X is halo, hydrogen, or a pharmaceutically acceptable, acid addition salt thereof ; which process comprises the reaction of a compound of the formula :

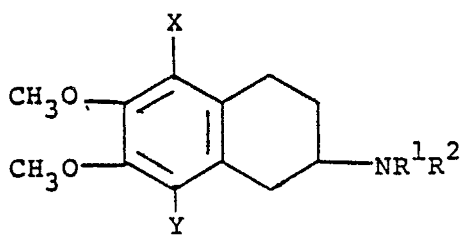

wherein X, Y, $R^1$ and $R^2$ are as defined above, with an ether splitting agent.

2. A process according to claim 1 for preparing a compound wherein X and Y are chosen from hydrogen, chlorine and fluorine.

3. A process according to claim 2 for preparing a compound wherein $R^1$ and $R^2$ are both hydrogen.

4. A process according to claim 3 for preparing a compound wherein X and Y are chosen from hydrogen and fluorine.

5. A process according to claim 2 for preparing a compound wherein one or both of $R^1$ and $R^2$ are other than hydrogen.

6. A process according to claim 1 for preparing the compound 8-chloro-6,7-dihydroxy-2-aminotetralin and pharmaceutically acceptable salts thereof.

7. A process according to claim 1 for preparing the compound 8-chloro-6,7-dihydroxy-2-[N-(n-propyl)-N-(4-hydroxyphenethyl)amino]tetralin and pharmaceutically acceptable salts thereof.

8. A process according to claim 1 for preparing a compound selected from the group comprising
8-fluoro-6,7-dihydroxy-2-[N-(n-propyl)-N-(4-hydroxyphenethyl)amino]tetralin ;
8-fluoro-6,7-dihydroxy-2-aminotetralin ;
8-chloro-6,7-dihydroxy-2-[N-(hydroxyphenethyl)amino]-tetralin ;

5-chloro-6,7-dihydroxy-2-aminotetralin ;
5-fluoro-6,7-dihydroxy-2-aminotetralin ;
and pharmaceutically acceptable salts thereof.

9. A process for the preparation of a pharmaceutical composition containing a compound as defined in any of claims 1 to 8, which process comprises bringing said compound into association with a pharmaceutically acceptable carrier.

10. A process according to any of claims 1 to 8 wherein the ether splitting agent is boron tribromide.

**Patentansprüche** (für die Vertragsstaaten : BE, CH, DE, FR, GB, IT, LI, LU, NL, SE)

1. Eine chemische Verbindung der Formel :

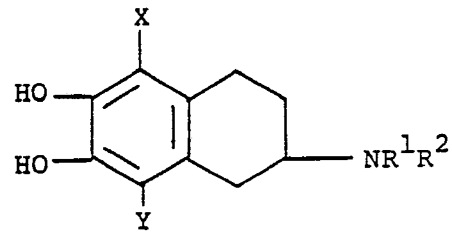

in der
$R^1$ und $R^2$ jeweils ein Wasserstoffatom, eine n-Propyl-, n-Butyl- oder 4-Hydroxyphenäthylgruppe bedeuten ;
X ein Wasserstoff- oder Halogenatom darstellt ; und
Y ein Halogenatom ist, wenn X ein Wasserstoffatom bedeutet, oder ein Wasserstoffatom ist, wenn X ein Halogenatom bedeutet, oder ein pharmazeutisch verträgliches Säureadditionssalz davon.

2. Verbindung nach Anspruch 1, in der X und Y Wasserstoff-, Chlor- oder Fluoratome sind.

3. Verbindung nach Anspruch 2, in der $R^1$ und $R^2$ beide Wasserstoffatome sind.

4. Verbindung nach Anspruch 3, in der X und Y Wasserstoff- oder Fluoratome sind.

5. Verbindung nach Anspruch 2, in der einer oder beide Reste $R^1$ und $R^2$ eine andere Bedeutung als Wasserstoffatom haben.

6. Verbindung nach Anspruch 1, nämlich 8-Chlor-6,7-dihydroxy-2-aminotetralin oder ein pharmazeutisch verträgliches Säureadditionssalz davon.

7. Verbindung nach Anspruch 1, nämlich 8-Chlor-6,7-dihydroxy-2-[N-(n-propyl)-N-(4-hydroxyphenäthyl)-amino]-tetralin oder ein pharmazeutisch verträgliches Säureadditionssalz davon.

8. Verbindung nach Anspruch aus der Gruppe :
8-Fluor-6,7-dihydroxy-2-[-(n-propyl)-N-(4-hydroxyphenäthyl)-amino]-tetralin ;
8-Fluor-6,7-dihydroxy-2-aminotetralin,
8-Chlor-6,7-dihydroxy-2-[N-(hydroxyphenäthyl)-amino]-tetralin ;
5-Chlor-6,7-dihydroxy-2-aminotetralin ;
5-Fluor-6,7-dihydroxy-2-aminotetralin ;
und pharmazeutisch verträgliche Säureadditionssalze davon.

9. Arzneimittel, umfassend eine Verbindung nach einem der Ansprüche 1 bis 8 und einen pharmazeutisch verträglichen Träger dafür.

10. Verbindung nach einem der Ansprüche 1 bis 8 zur Verwendung als dopaminerger Wirkstoff.

11. Verfahren zur Herstellung einer Verbindung nach einem der Ansprüche 1 bis 8, umfassend die Umsetzung einer Verbindung der Formel (II)

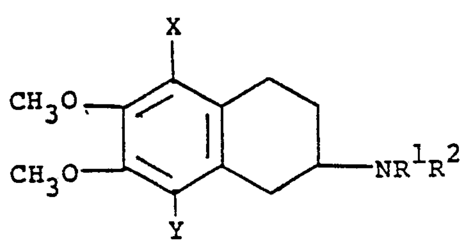

(II)

in der X, Y, $R^1$ und $R^2$ wie in Anspruch 1 definiert sind, mit einem ätherspaltenden Agens.

**Patentansprüche** (für den Vertragsstaat AT)

1. Ein Verfahren zur Herstellung einer Verbindung der Formel :

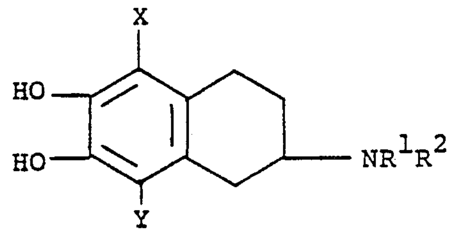

in der

R¹ und R² jeweils ein Wasserstoffatom, eine n-Propyl-, n-Butyl- oder 4-Hydroxyphenäthylgruppe bedeuten ;

X ein Wasserstoff- oder Halogenatom darstellt ; und

Y ein Halogenatom ist, wenn X ein Wasserstoffatom bedeutet, oder ein Wasserstoffatom ist, wenn X ein Halogenatom bedeutet, oder eines pharmazeutisch verträglichen Säureadditionssalzes davon, umfassend die Umsetzung einer Verbindung der Formel (II)

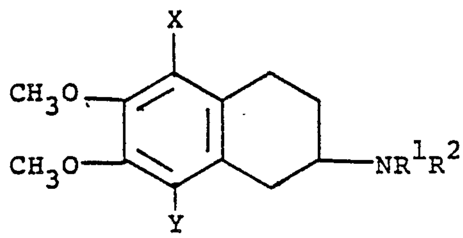

in der X, Y, $R^1$ und $R^2$ wie vorstehend definiert sind, mit einem ätherspaltenden Agens.

2. Verfahren nach Anspruch 1 zur Herstellung einer Verbindung, in der X und Y Wasserstoff-, Chlor- oder Fluoratome sind.

3. Verfahren nach Anspruch 2 zur Herstellung einer Verbindung, in der $R^1$ und $R^2$ beide Wasserstoffatome sind.

4. Verfahren nach Anspruch 3 zur Herstellung einer Verbindung, in der X und Y Wasserstoff- oder Fluoratome sind.

5. Verfahren nach Anspruch 2 zur Herstellung einer Verbindung, in der einer oder beide Reste $R^1$ und $R^2$ eine andere Bedeutung als Wasserstoffatom haben.

6. Verfahren nach Anspruch 1 zur Herstellung der Verbindung 8-Chlor-6,7-dihydroxy-2-aminotetralin oder pharmazeutisch verträglichen Säureadditionssalzen davon.

7. Verfahren nach Anspruch 1 zur Herstellung der Verbindung 8-Chlor-6,7-dihydroxy-2-[N-(n-propyl)-N-(4-hydroxyphenäthyl)-amino]-tetralin oder pharmazeutisch verträglichen Säureadditionssalzen davon.

8. Verfahren nach Anspruch 1 zur Herstellung einer Verbindung aus der Gruppe :

8-Fluor-6,7-dihydroxy-2-[-(n-propyl)-N-(4-hydroxyphenäthyl)-amino]-tetralin ;
8-Fluor-6,7-dihydroxy-2-aminotetralin ;
8-Chlor-6,7-dihydroxy-2-[N-(hydroxyphenäthyl)-amino]-tetralin ;
5-Chlor-6,7-dihydroxy-2-aminotetralin ;
5-Fluor-6,7-dihydroxy-2-aminotetralin ;

und pharmazeutisch verträglichen Säureadditionssalzen davon.

9. Verfahren zur Herstellung eines Arzneimittels, das eine Verbindung nach einem der Ansprüche 1 bis 8 enthält durch Zusammenbringen der Verbindung mit einem pharmazeutisch verträglichen Träger.

10. Verfahren nach einem der Ansprüche 1 bis 8, in dem das ätherspaltende Agens Bortribromid ist.

**Revendications** (pour les Etats Contractants : BE, CH, DE, FR, GB, IT, LI, LU, NL, SE)

1. Un composé chimique de la formule :

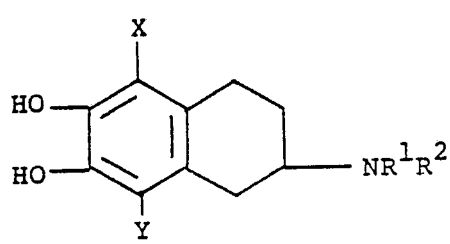

dans laquelle :

$R^1$ et $R^2$ sont, chacun, hydrogène, n-propyle, n-butyle ou 4-hydroxyphénéthyle ;

X est hydrogène ou halo ; et

Y est, lorsque X est hydrogène, halo ou, lorsque X est halo, hydrogène, ou un sel d'addition acide du même pharmaceutiquement acceptable.

2. Un composé selon la revendication 1 dans lequel X et Y sont choisis parmi l'hydrogène, le chlore ou le fluor.

3. Un composé selon la revendication 2 dans lequel $R^1$ et $R^2$ sont tous les deux de l'hydrogène.

4. Un composé selon la revendication 3 dans lequel X et Y sont choisis entre l'hydrogène et le fluor.

5. Un composé selon la revendication 2 dans lequel l'un ou les deux $R^1$ et $R^2$ sont différents de l'hydrogène.

6. Un composé selon la revendication 1 qui est : 8-chloro-6,7-dihydroxy-2-aminotétraline ou un sel d'addition acide du même pharmaceutiquement acceptable.

7. Un composé selon la revendication 1 qui est : 8-chloro-6,7-dihydroxy-2-[N-(n-propyle)-N-(4-hydroxyphénéthyle) amino] tétraline ou un sel d'addition acide du même pharmaceutiquement acceptable.

8. Un composé selon la revendication 1 qui est choisi dans le groupe comprenant :

8-fluoro-6,7-dihydroxy-2-[N-(n-propyle)-N-(4-hydroxyphénéthyle) amino] tétraline ;

8-fluoro-6,7-dihydroxy-2-aminotétraline ;

8-chloro-6,7-dihydroxy-2-[N-hydroxyphénéthyle amino]-tétraline ;

5-chloro-6,7-dihydroxy-2-aminotétraline ;

5-fluoro-6,7-dihydroxy-2-aminotétraline ;

et les sels d'addition acides du même pharmaceutiquement acceptables.

9. Une composition pharmaceutique comprenant un composé comme défini dans n'importe laquelle des revendications de 1 à 8 et un support pharmaceutiquement acceptable pour le même.

10. Un composé selon n'importe laquelle des revendications de 1 à 8 à employer en tant qu'agent dopaminergique.

11. Un procédé pour la préparation d'un composé comme défini dans n'importe laquelle des revendications de 1 à 8, procédé qui comprend la réaction d'un composé de la formule (II) :

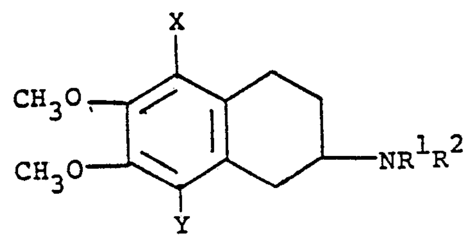

(II)

dans laquelle X, Y, $R^1$ et $R^2$ sont comme définis dans la revendication 1, avec un agent de cission pour l'éther.

**Revendications** (pour l'Etat contractant AT)

1. Un procédé pour la préparation de la formule :

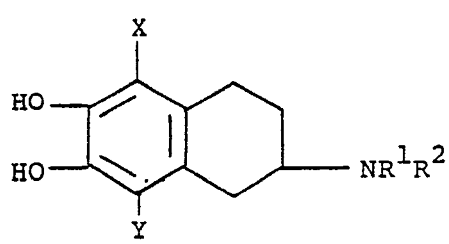

dans lequel :

$R^1$ et $R^2$ sont, chacun, hydrogène, n-propyle, n-butyle ou 4-hydroxyphénéthyle ;

X est hydrogène ou halo ; et

Y est, lorsque X est hydrogène, halo ou, lorsque X est halo, hydrogène, ou un sel d'addition acide du même pharmaceutiquement acceptable ; procédé qui comprend la réaction d'un composé de la formule :

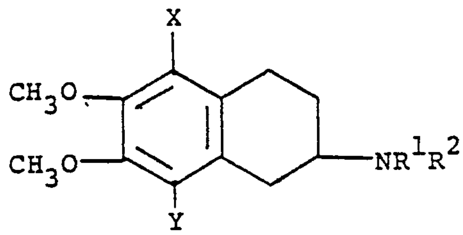

dans laquelle X, Y, $R^1$ et $R^2$ sont comme définis ci-dessus, avec un agent de cission de l'éther.

2. Un procédé selon la revendication 1 pour la préparation d'un composé dans lequel X et Y sont choisis parmi l'hydrogène, le chlore et le fluor.

3. Un procédé selon la revendication 2 pour la préparation d'un composé dans lequel $R^1$ et $R^2$ sont tous deux de l'hydrogène.

4. Un procédé selon la revendication 3 pour la préparation d'un composé dans lequel X et Y sont choisis parmi l'hydrogène et le fluor.

5. Un procédé selon la revendication 2 pour la préparation d'un composé dans lequel l'un ou les deux $R^1$ et $R^2$ sont différents de l'hydrogène.

6. Un procédé selon la revendication 1 pour la préparation du composé 8-chloro-6,7-dihydroxy-2-aminotétraline et les sels du même pharmaceutiquement acceptables.

7. Un procédé selon la revendication 1 pour la préparation du composé 8-chloro-6,7-dihydroxy-2-[N-(n-propyle)-N-(4-hydroxyphénéthyle) amino] tétraline et les sels du même pharmaceutiquement acceptables.

8. Un procédé selon la revendication 1 pour la préparation d'un composé choisi parmi le groupe comprenant :

8-fluoro-6,7-dihydroxy-2-[N-(n-propyle)-N-(4-hydroxyphénéthyle) amino] tétraline ;
8-fluoro-6,7-dihydroxy-2-aminotétraline ;
8-chloro-6,7-dihydroxy-2-[N-hydroxyphénéthyle amino]-tétraline ;
5-chloro-6,7-dihydroxy-2-aminotétraline ;
5-fluoro-6,7-dihydroxy-2-aminotétraline ;

et les sels du même pharmaceutiquement acceptables.

9. Un procédé pour la préparation d'une composition pharmaceutique contenant un composé comme défini dans n'importe laquelle des revendications de 1 à 8, procédé qui comprend la mise en association dudit composé avec un support pharmaceutiquement acceptable.

10. Un procédé selon n'importe laquelle des revendications de 1 à 8 dans lequel l'agent de cission de l'éther est le tribromure de bore.